# EUROPEAN PATENT APPLICATION

(11) **EP 4 607 529 A1**
(43) Date of publication of application: **27.08.2025**
(21) Application number: 25158276.3
(22) Date of filing: 17.02.2025
(51) Int. Cl.: G16H 30/40, G06N 3/02, G06T 7/00, G16H 50/20, G16H 50/30, G16H 50/70

(54) **VULNERABLE PLAQUE ASSESSMENT AND OUTCOME PREDICTION IN CORONARY ARTERY DISEASE**

(30) Priority: 20.02.2024 US 202418581430; 20.02.2024 EP 24465507
(71) Applicant: Siemens Healthineers AG, 91301 Forchheim (DE)
(72) Inventor: TURCEA, Alexandru, 105500 Busteni, Prahova (RO); ITU, Lucian Mihai, 500294 Brasov (RO); SHARMA, Puneet, Princeton Junction, NJ, 08550 (US); CIMEN, Serkan, West Orange, NJ, 07052 (US); NEUMANN, Dominik, 91052 Erlangen (DE)
(74) Representative: HKW Intellectual Property PartG mbB

(57) **Abstract**

Systems and methods for vulnerable plaque assessment and outcome prediction in coronary artery disease. Medical imaging data is used to generate a coronary tree model (300) of coronary centerlines (310) of a patient (210). The coronary tree model (300) includes a plurality of nodes that represent locations in the coronary tree model (300). Feature embedding associated with each node are determined from a plurality of features derived from the medical imaging data. The feature embeddings are input into a trained graph neural network that is configured to output an assessment at a node level, a segment level, and/or a coronary tree level for vulnerable plaque.

## Description

### FIELD

This disclosure relates to medical imaging and assessing coronary artery disease.

### BACKGROUND

Coronary artery disease is a common heart condition where the major blood vessels that supply the heart (coronary arteries) struggle to send enough blood, oxygen, and nutrients to the heart muscle. Coronary artery disease starts when fats, cholesterols and other substances collect on the inner walls of the heart arteries forming plaque. This condition is called atherosclerosis. This plaque can cause the arteries to narrow, blocking blood flow. The plaque can also burst, leading to a blood clot.

Coronary artery stenosis is a narrowing of coronary lumen space caused by an atherosclerotic lesion. Treatment for coronary artery disease usually involves lifestyle changes such as not smoking, eating healthy and exercising more. Sometimes, medications and procedures are needed. The main goals of coronary artery stenoses treatment are: (i) to improve the patient's quality of life and (ii) to minimize the risk of future cardiac events. The treatment decision is primarily based on the functional or anatomical significance of the stenosis. In general, a type of plaque in the coronary arteries that is more likely to rupture or cause a blood clot is referred to as 'vulnerable plaque'. Vulnerable plaques may be present both in significant and non-significant coronary lesions. There is a subset of stenoses which are functionally and / or anatomically not significant but are likely to cause MACE (major adverse cardiovascular events). As such, functional or anatomical significance is not the sole determinant of future adverse events. A recent prospective study even suggests that non-flow-limiting lesions with vulnerable characteristics are responsible for the majority of adverse events during follow-up. Accurately identifying coronary plaque vulnerability may lead to better medical diagnostics and may benefit a patient's health outcomes.

### SUMMARY

By way of introduction, the preferred embodiments described below include methods, systems, instructions, and computer readable media for assessing coronary plaque vulnerability using a graph neural network based on coronary tree-level data and patient-level data. The invention is defined by the attached set of claims. Further details of the disclosed method, devices and system are described below, which are helpful for understanding the claimed invention.

In a first aspect, a method is provided for vulnerable plaque assessment and outcome prediction in coronary artery disease. The method includes: acquiring medical imaging data of a patient; generating a coronary tree model of coronary centerlines of the patient from the medical imaging data, the coronary tree model comprising a plurality of nodes that represent locations in the coronary tree model; determining a feature embedding associated with each node from a plurality of features derived from the medical imaging data; inputting the feature embeddings into a graph neural network; and outputting an assessment at a node level, a segment level, and/or a coronary tree level for vulnerable plaque based on the output of the graph neural network.

The medical imaging data may be coronary computed tomography angiography (CCTA) data. In an embodiment, the method further includes acquiring patient level data, wherein the patient level data is not specific to any location in the coronary tree model, wherein the patient level data is input into the graph neural network. The patient level data comprises at least one of a presence of other pathologies linked to coronary artery disease, patient demographics, patient history, family history, a calcium scored, an overall plaque burden, lab results, or results of a stress test.

In an embodiment, generating the coronary tree model comprises segmenting the medical imaging data using a thresholding method, wherein the coronary tree model includes all locations with a diameter larger than a given threshold of 1.0 mm. Generating the coronary tree model may comprise deriving a 2.5D geometry by matching 2D geometries extracted from each acquisition.

In an embodiment, determining the feature embeddings comprises: defining a set of features fi(x,t) for each respective node of the plurality of nodes, where fi refers to the feature, x refers to a 3D location in the coronary tree model, and t refers to a time; and inputting the set of features into a machine trained network configured to output a feature embedding for each respective node. Values for the set of features may change depending on a state of the patient. Multiple instances of certain features are averaged to determine the set of features.

**In** an embodiment, the graph neural network uses a message-passing mechanism to aggregate, process and pass information between the nodes of the graph neural network . Virtual edges connecting nodes pertaining to the same coronary segment are added to the graph neural network for the message-passing mechanism.

In an embodiment, the assessment comprises a risk score defined at lesion or patient level, assessing the risk of rupture, clot formation, or erosion over a certain time frame. The method may further includes assessing the vulnerable plaque of the patient based on the assessment and providing procedural indications that allow for minimizing a risk to the patient of the vulnerable plaque. In an embodiment, the vulnerable plaque of the patient is assessed after the medical imaging data is acquired, wherein the assessment is updated during a cathlab exam, and wherein the assessment is further updated after the cathlab exam.

**In** a second aspect, a system is provided for vulnerable plaque assessment. The system includes a medical imaging system, an image processing system, and an output interface. The medical imaging system is configured to acquire medical imaging data of a patient. The image processing system is configured to generate a coronary tree of coronary centerlines of a patient from the medical imaging data, the coronary tree model comprising a plurality of nodes that represent locations in the coronary tree model, the image processing system further configured to compute or derive one or more features associated with each node from a plurality of features derived from the medical imaging data, the image processing system further configured to determine a feature embedding for each node based on the one or more features and input the feature embeddings into a graph neural network configured to generate a vulnerable plaque assessment. The output interface is configured to provide the vulnerable plaque assessment.

**In** a third aspect, a non-transitory computer implemented storage medium that stores machine-readable instructions executable by at least one processor is provided. The machine-readable instructions include generating a coronary tree model of coronary centerlines of a patient from medical imaging data, the coronary tree model comprising a plurality of nodes that represent locations in the coronary tree model; determining a feature embedding associated with each node from a plurality of features derived from the medical imaging data; inputting the feature embeddings into a graph neural network; and outputting an assessment at a node level, a segment level, and/or a coronary tree level for vulnerable plaque based on the output of the graph neural network.

Any one or more of the aspects described above may be used alone or in combination. In particular, features described with respect to the method according to the first aspect are also applicable to the system according to the second aspect and the computer implemented storage medium according to the third aspect, or vice versa. These and other aspects, features and advantages will become apparent from the following detailed description of preferred embodiments, which is to be read in connection with the accompanying drawings. The present invention is defined by the following claims, and nothing in this section should be taken as a limitation on those claims. Further aspects and advantages of the invention are discussed below in conjunction with the preferred embodiments and may be later claimed independently or in combination.

### BRIEF DESCRIPTION OF THE DRAWINGS

Figure 1 depicts an example system for assessing coronary plaque vulnerability according to an embodiment.
Figure 2 depicts an example CT scanning system.
Figure 3 depicts an example of a 3D centerline tree structure.
Figure 4 depicts an example method for assessing coronary plaque vulnerability according to an embodiment.

### DETAILED DESCRIPTION

Embodiments described herein provide systems and methods for assessing coronary plaque vulnerability at lesion level and the risk of a major adverse clinical event (MACE) at patient level. Two types of input data are used including Coronary tree-level data and Patient-level data. A model of the coronary centerlines of patient is generated and populated using feature data (coronary tree-level data). Patient characteristics and demographics (patient-level data) is also acquired. A graphnet architecture is used to predict vulnerable plague indicators, for example hemodynamic measures, QCA scores, and plaque vulnerability scores. The predictions / assessment may be used for providing or supporting a clinical decision for the patient.

As used herein, 'vulnerable plaque' refers to a type of plaque in the coronary arteries that is more likely to rupture or cause a blood clot, for example, that are prone to cause a coronary event, either by rupture or erosion causing acute thrombosis, or by rapid plaque progression leading to significant stenosis and subsequent flow limitation. Vulnerable plaque characteristics have been defined in previous studies and include features such as spotty calcification, thin cap fibroatheroma, napkin-ring sign, eccentricity, and low attenuation plaque among others. These aspects however define only properties indicative of a higher risk of rupture, and do not allow for an accurate identification of plaques that are likely to rupture. Assessment of plain angiography images may better inform an operator about lesion specifics such as the presence of calcifications, thrombosis, or the extent of luminal obstruction as expressed in percentage of diameter stenosis. However, morphological characteristics of plaques cannot typically be distinguished without the use of intracoronary imaging such as IVUS or OCT and state-of-the-art CT systems (PCCT). IVUS is an invasive imaging technique that uses ultrasound to visualize the inside of the coronary artery walls. The technique uses ultrasonography, where high-energy sound waves are radiated into the tissue, and the reflection is returned to the transducer and converted into images. OCT uses coherent near infrared light to generate images by measuring the intensity of light returning from the vessel wall. However, the application of invasive functional and morphological assessments is restricted by equipment availability, financial considerations, as well as prolonged procedural duration. The accurate identification of a "true" vulnerable plaque procedure from more generally available information would potentially allow for a superior risk stratification in patients with coronary artery disease.

Embodiments provide systems and methods that implement efficient deep learning-based methods, e.g., a graph neural network, by organizing all relevant data for vulnerable plaque assessment and outcome prediction in coronary artery disease into a tree like structure. The disclosed embodiments may be implemented to computationally facilitate processing of medical imaging data and consequently improving and optimizing medical diagnostics. Embodiments leverage the power of artificial intelligence (AI), for example using a graph neural network to improve the assessment of vulnerable plague. The systems and methods provide automatic interpretation of imaging data thus streamlining the diagnostic process. The use of the described methods and systems further limits errors by removing user errors and decisions from the process.

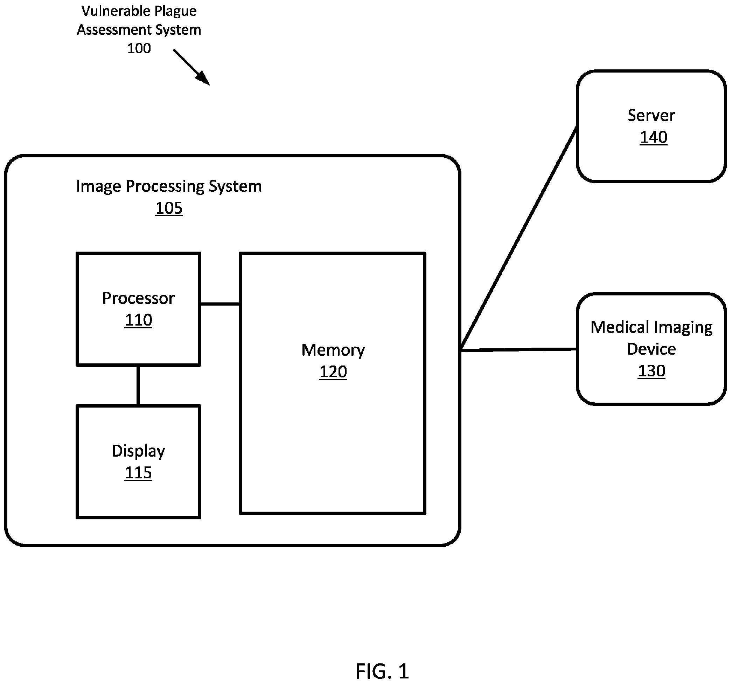

Figure 1 depicts a system 100 for assessing coronary plaque vulnerability. The system 100 includes a medical imaging device 130 and an image processing system 105. A server 140 may be provided that stores or processes data, for example using a cloud based system. The medical imaging device 130 acquires medical imaging data. The image processing system 105 uses a graph neural network to assess coronary plaque vulnerability. The graph neural network is configured to input a coronary tree model that includes a plurality of nodes and edges, where the nodes represent locations in the coronary tree and include a set of features fi(x,t) where fi refers to the feature, x refers to the 3D/2D location, and t refers to the time. The image processing system 105 determines feature embedding associated with each node from patient-level data and the respective set of features for the respective node. The feature embeddings are input into the graph neural network which outputs diagnostic information at a node level, a segment level, and/or a coronary tree level.

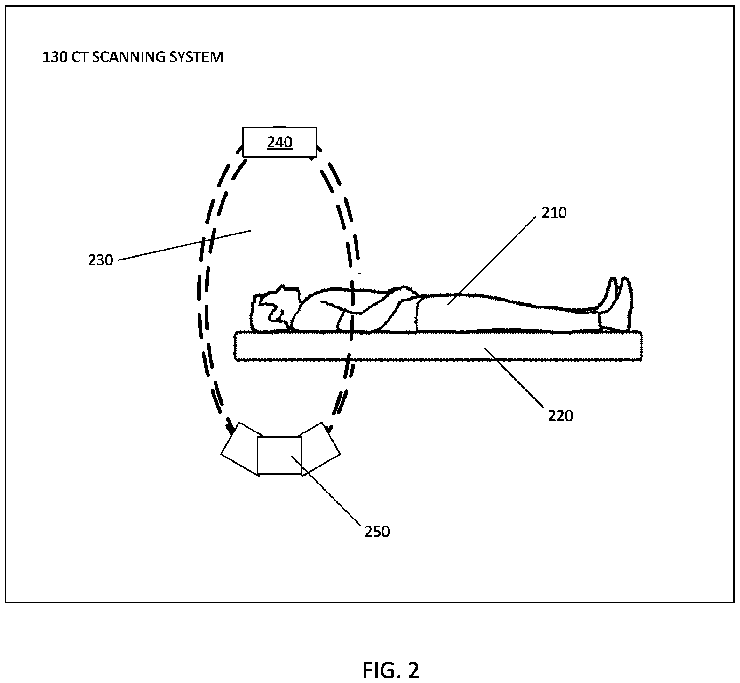

In an example, the medical imaging device 130 performs computed tomography (CT) to produce image data that is used by the system. Other types of scanners may be used (e.g., MR, PET, SPECT, or other medical imaging devices). The CT scanning device is only exemplary, and a variety of CT scanning systems can be used to collect the CT data. Figure 2 depicts an example CT imaging system 130. An object 210 (e.g., a patient 210) is positioned on a table 220 that is configured, via a motorized system, to move the table 220 to multiple positions through a circular opening 230 in the CT imaging system 130. An X-ray source 240 (or other radiation source) and detector element(s) 250 are a part of the CT imaging system 130 and are configured to rotate around the subject 210 on a gantry while the subject is inside the opening / bore 230. The rotation may be combined with movement of the bed to scan along a longitudinal extent of the patient 210. Alternatively, the gantry moves the source 240 and detector 250 in a helical path about the patient 210. In a CT imaging system 130, a single rotation may take approximately one second or less. During the rotation of the X-ray source 240 and/or detector, the X-ray source 240 produces a narrow, fan-shaped (or cone-shaped) beam of X-rays that pass through a targeted section of the body of the subject 210 being imaged. The detector element(s) 250 (e.g., multi-ring detector elements) are opposite the X-ray source 240 and register the X-rays that pass through the body of the subject being imaged and, in that process, record a snapshot used to create an image. Many different snapshots at many angles through the subject are collected through one or more rotations of the X-ray source 240 and/or detector element(s) 250. The image data generated by the collected snapshots are transmitted to a control unit that stores or processes the image data based on the snapshots into one or several cross-sectional images or volumes of an interior of the body (e.g., internal organs or tissues) of the subject being scanned by the CT imaging system 130. Any now known or later developed CT system may be used. Other x-ray scanners, such as a CT-like C-arm scanner, may be used.

The medical imaging device 130 is configured to generate imaging data or medical images of a patient 110. The imaging data or the medical image is data representing a two-dimensional slice or a three-dimensional volume of the subject. The data may be in any format. The three-dimensional representation may be formatted as a stack or plurality of two-dimensional planes or slices. Values are provided for each of multiple locations distributed in two or three dimensions. The medical imaging data is acquired as one or more frames of data. The frame of data represents the scan region at a given time or period. The dataset may represent the area or volume over time, such as providing a 4D representation of the subject. While the terms image and imaging are used, the image or imaging data may be in a format prior to actual display of the image. For example, the medical imaging data may be a plurality of scalar values representing different locations in a Cartesian or polar coordinate format different than a display format. As another example, the medical image may be a plurality of red, green, blue (e.g., RGB) values output to a display for generating the image in the display format. The medical image may be currently or previously displayed image in the display or another format. The imaging data is a dataset that may be used for imaging, such as scan data or a generated image representing a portion of the patient.

The medical imaging data or medical image is processed by the image processing system 105. The image processing system 105 includes a processor 110, display 115, and memory 120. The image processing system 105 may receive or transmit data to and from the server 140 that may also be configured to process the image or store data for future image processing or training / storage of machine trained models. The image processing system 105 is configured to input the imaging data and output a 3D centerline coronary tree structure and, at each location of the tree, a set of features. The image processing system 105 is further configured to determine a feature embedding at each node. The image processing system 105 is further configured to input the feature embeddings and coronary tree structure into a trained graph neural network that is configured to output a prediction of vulnerable plaque.

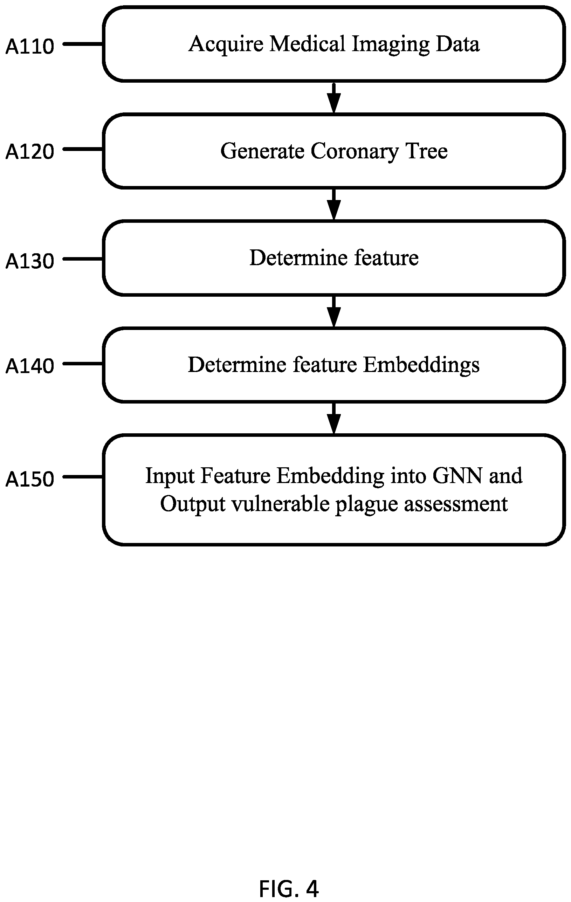

The processor 110 is a general processor, digital signal processor, graphics processing unit, application specific integrated circuit, field programmable gate array, artificial intelligence processor, digital circuit, analog circuit, combinations thereof, or other now known or later developed device for processing images, normalizing image data, registering image data, augmenting image data, among other steps described below. The processor is a single device, a plurality of devices, or a network. For more than one device, parallel or sequential division of processing may be used. Different devices making up the processor 110 may perform different functions. In one embodiment, the processor 110 is a control processor or other processor of the medical imaging device 130. In other embodiments, the processor 110 is part of a separate workstation or computer. The processor 110 operates pursuant to stored instructions to perform various acts described herein. The processor 110 is configured by software, design, firmware, and/or hardware to perform any or all of the acts of Figure 4.

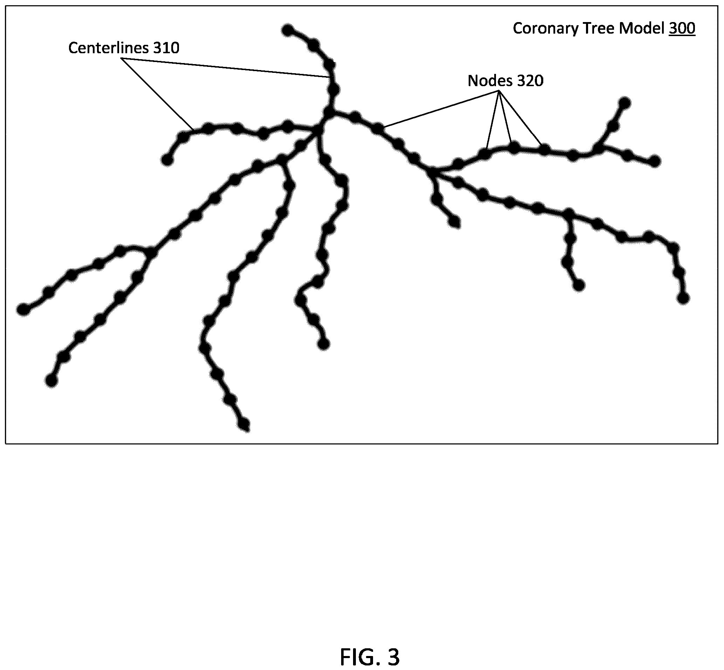

The image processing system 105 is configured to input the imaging data and output a 3D centerline coronary tree structure. Figure 3 depicts an example of a 3D centerline tree structure 300 made up of centerlines 310 and nodes 320. At each node 320, a set of features Fi(x, t) are provided. In an embodiment, the 3D model of the coronary centerlines includes all locations with a diameter larger than a given threshold 1.0 or 1.5 mm. The coronary tree model 300 may be reconstructed from coronary computed tomography angiography (CCTA) (for example, Photon-Counting Computed Tomography (PCCT) or regular CTA) or from coronary angiography data. Vessel centerlines may be extracted, for example, by segmentation and a thinning pipeline or by direct tracking. In an embodiment, a convolutional neural network (CNN) is provided for automatic centerline extraction. Any method may be used to generate the 3D coronary tree model 300 from the imaging data or other acquired data. In an embodiment, the anatomical structure is determined by the image processing system 105, for example, by identifying the locations of the vessels in the acquired CT data. Any now known or later developed approach to determine the anatomical structure may be used. For example, an adaptive region growing and skeletonization approach is used. One or more seeds, such as those in/for the vessel, are located by the processor or manual entry. The seed or seeds are used in region growing to find locations of the vessel tree. Skeletonization may be used to model the vessel structure, such as using lines to represent the centers of the branches of the vessel tree. Using threshold, random walker, or other approach, the vessels and the coronary tree model 300 of the coronary centerlines may be segmented for calculating anatomical features. A generic model of the coronary tree model 300 of the coronary centerlines may be fit to the segmented anatomy or to locations identified as vessel. Any fitting may be used.

The coronary tree model 300 is based on individual centerline points as depicted in the Figure 3. At each location / node 320 a set of features fi(x,t) are defined by the image processing system 105 where fi refers to the feature, x refers to the 3D location, and t refers to the time. In an embodiment, instead of using a 3D geometry reconstructed from multiple coronary angiography acquisitions, a 2.5D geometry may be derived by matching 2D geometries extracted from each acquisition. In this case, each feature fi may either have multiple instances, i.e. one instance for each acquisition, or a unique feature value fi(x,t) may be derived by aggregating the matched corresponding features from each view. Alternatively, the features fi may be expressed as a probability distribution derived from information extracted from multiple acquisitions. This may also allow for quantifying the uncertainty of the predicted results.

Certain features may be constant over time. Others may change over time. Certain features may be obtained by using AI models or algorithms on the available input data. The values of the features may change depending on the state of the patient (rest, exercise - hyperemia, intermediate state). Hence, multiple instances of these features may be available. Derived features like percentage diameter stenosis, wall strain (min / max), time-averaged WSS may also be included. These have been shown to be linked with vulnerable plaque in the past. Multiple image acquisitions depicting the same coronary tree or part of a coronary tree may be available when employing angiography and / or CCTA. Hence, multiple instances of certain features may be available. These may be provided either independently or compounded, e.g., through averaging.

A list of potentially relevant features includes the following features described below. Additional or fewer features may be used. Not all features described below may be used. Whether or not values for certain features are computed may be determined based on the input data and computational resources. After configuring and training the model (described below), certain features may be cut due to processing time / resources or based on their impact on the output of the model. Potential features include:
r(x,t): time-varying lumen radius at each centerline location.
rref(x,t): time-varying reference (i.e. healthy) lumen radius at each centerline location.
router_wall(x,t): time-varying outer wall radius at each centerline location. It allows for the assessment of positive remodeling. Vulnerable plaque can cause outward bulging of the artery wall, which is known as positive remodeling. This can be seen as an increase in the diameter of the artery segment containing the plaque compared to normal adjacent segments.
curvature(x,t): time-varying curvature value at each centerline location.
stent(x): binary variable specifying whether a stent is present at each centerline location. Alternatively, an integer type may be used since stents may overlap, and at a given centerline location multiple stents may be present.
label(x): integer variable specifying the coronary segment to which the current location pertains.
stenosis(x): may be an integer variable specifying a unique id of the stenosis or a floating point variable specifying the stenosis probability at this location.
contrast(x,t): integer variable specifying the contrast intensity at this location, which typically varies in time.
CABG(x): binary variable specifying whether each centerline location is part of a bypass graft.
collateral(x): binary variable specifying whether each centerline location is part of a collateral artery.
CTO(x): binary variable specifying if a CTO is encountered at a centerline location (typically a leaf node).
myocardialBridging(x): binary variable specifying whether myocardial bridging is observed at a centerline location. Additional similar variables may be defined for other coronary anomalies (e.g., aneurysm).
artefact(x): binary variable specifying whether an image artefact is observed at a centerline location (e.g., motion artefact, vessel overlap, etc.).
angulation(x): floating point variable specifying the bifurcation angulation at a centerline location (0 if no bifurcation is present).
plaqueType(x): integer variable specifying the plaque type: fibrous, fatty, calcified, mixed, etc. Such information may be extracted from angiography (limited) or from CCTA (rich).
materialProperties(x): in case PCCT is available, the multi-energy capabilities of PCCT are exploited, which allow for a better characterization of tissue properties and material composition.
radiomicFeatures(x)
plaquePattern(x): integer variable describing the presence of various plaque patterns indicative of vulnerable plaques. These patterns may include spotty calcification where vulnerable plaque may have small areas of calcification within the plaque, which appear as small, discrete spots on CT images, thin cap fibroatheroma; a Napkin-ring sign, where the napkin-ring sign is a feature seen on CT images that may be indicative of vulnerable plaque. The Napkin-ring sign appears as a ring of high attenuation around a low attenuation core, resembling a napkin ring. This sign suggests that the plaque has a large lipid-rich core and a thin fibrous cap. Eccentricity includes where vulnerable plaque often has an irregular or eccentric shape, with the plaque material located mainly on one side of the vessel wall. Low attenuation plaque includes where vulnerable plaque typically has a large lipid-rich core, which has a lower attenuation value on CT images compared to surrounding tissue. This can appear as a soft, low-density area within the wall of the artery.
regionalWallMotion(x): may be derived from angiography, echocardiography, or multi-phase CCTA.
downstreamMyocardialVolume(x): myocardial volume subtended by the coronary tree downstream from a given location x.

Computed features may also include:
FFR(x), IFR(x), restPdPa(x), etc : hemodynamic features computed or predicted for the coronary tree.
WSS(x,t), OSI(x,t): location and time-dependent wall shear stress, oscillatory shear index. Previous studies have shown the high / low stress are linked with plaque evolution and or rupture.
Q(x,t): time-varying flow rate.
V(x,t): time-varying velocity.

The features are extracted / computed automatically with the image processing system 105, the processor, or semi-automatically, for example, with the processor and user input. Under a fully automated approach, an underlying image processing algorithm detects the features or values by filtering, thresholding, pattern matching, and/or other image processing. The features are extracted by the processor from the medical image data representing the detected regions. This automatic approach occurs without user input of locations of the anatomy. Under a semi-automated approach, some of the features may be extracted automatically by an algorithm, while some others may be annotated or edited/corrected by a human. In another example, the user places calipers or other measuring tools, and the processor determines the value of the feature from the calipers. In alternative approaches, one or more features are extracted manually. The user views an image and manually determines the value of the feature. The user enters an annotation or fills in a field with the value of the manually determined feature.

In an embodiment, the features of the plaque and/or the vessel are extracted from the medical image data using one or more machine learning (ML) trained models. For example, the processor or other device extracts the shape, geometry, inlet angle, outlet angle, location of the plaque relative to the vessel, stenosis of the vessel, vessel size, vessel shape, or combinations thereof using a specifically trained model. Other features may be extracted or derived therefrom. For example, anatomic features of the plaque, such as the shape, geometry, inlet and outlet angles, location (e.g., bifurcation or otherwise) may be extracted from segmented or processed CT angiography images.

In an embodiment, the machine learned network(s) or model(s) used to extract feature data include a neural network that is defined as a plurality of sequential feature units or layers. Sequential is used to indicate the general flow of output feature values from one layer to input to a next layer. Sequential is used to indicate the general flow of output feature values from one layer to input to a next layer. The information from the next layer is fed to a next layer, and so on until the final output. The layers may only feed forward or may be bi-directional, including some feedback to a previous layer. The nodes of each layer or unit may connect with all or only a sub-set of nodes of a previous and/or subsequent layer or unit. Skip connections may be used, such as a layer outputting to the sequentially next layer as well as other layers. Rather than pre-programming the features and trying to relate the features to attributes, the deep architecture is defined to learn the features at different levels of abstraction based on the input data. The features are learned to reconstruct lower-level features (i.e., features at a more abstract or compressed level). Each node of the unit represents a feature. Different units are provided for learning different features. Various units or layers may be used, such as convolutional, pooling (e.g., max pooling), deconvolutional, fully connected, or other types of layers. Within a unit or layer, any number of nodes is provided. For example, 100 nodes are provided. Later or subsequent units may have more, fewer, or the same number of nodes.

The processor 110 is configured to train the model(s) using a machine training method and training data. The training data may be acquired at any point prior to inputting the training data into the model. Different models may be configured for different tasks, for example, different models for determining each of the relevant features. Certain models may be used for computing multiple of the relevant features. The output of certain models may be used by other models for determining certain relevant features. For example, one machine trained model may perform segmentation while another may use the output of the segmentation to derive values or provide classification for a particular relevant features. For training and applying a machine trained model there are two stages, a training stage for generating or training the model using a collection of training data and an application stage for applying the generated / trained entity matching network to new unseen (unlabeled) data. The training stage includes acquiring training data during patient scans, processing the training data, and inputting the training data into the model in order to generate a trained model. The output is a trained model that is applied in the application stage. The application stage includes receiving real-time data from, for example, a CT scan, and applying the trained model that was trained during the training stage to compute values for a respective relevant feature. The training stage may be performed at any point prior to the application stage. The training stage may be repeated after new training data is acquired. The application stage may be performed at any point after the training stage generates the trained network and real-time data is received.

The machine learned network(s) and other data may be stored in a memory 120 alone with the instructions for implementing the processes, methods, and/or techniques discussed herein are provided on non-transitory computer-readable storage media or memories, such as a cache, buffer, RAM, removable media, hard drive, or other computer readable storage media. The instructions are executable by the processor or another processor. Computer readable storage media include various types of volatile and nonvolatile storage media. The functions, acts or tasks illustrated in the figures or described herein are executed in response to one or more sets of instructions stored in or on computer readable storage media. The functions, acts or tasks are independent of the instructions set, storage media, processor or processing strategy and may be performed by software, hardware, integrated circuits, firmware, micro code, and the like, operating alone or in combination. In one embodiment, the instructions are stored on a removable media device for reading by local or remote systems. In other embodiments, the instructions are stored in a remote location for transfer through a computer network. In yet other embodiments, the instructions are stored within a given computer, CPU, GPU, or system. Because some of the constituent system components and method steps depicted in the accompanying figures may be implemented in software, the actual connections between the system components (or the process steps) may differ depending upon the manner in which the present embodiments are programmed.

In addition to the relevant feature data, the system 100 is configured to acquire or otherwise use patient-level data. Patient-level data is not linked to a specific location in the coronary tree, rather the patient-level data refers to properties of the patient such as the presence of other pathologies linked to coronary artery disease: AV stenosis, etc., the patient's demographics, and the patient's medical history and family history. The patient level data may further include an overall plaque burden, for example the amount of plaque in the coronary arteries. Higher levels of plaque burden may indicate the presence of vulnerable plaque. Lab results for the patient, in particular markers related to inflammation may be used as inflammation plays a key role in the development and progression of vulnerable plaques. Elevated levels of inflammatory markers such as C-reactive protein (CRP) may indicate the presence of vulnerable plaque. Stress tests for the patient may also be used. For example, a positive stress test may suggest the presence of vulnerable plaque in the coronary arteries. The patient level data may be acquired automatically or semi automatically. For example, the system may parse or search a patient's medical history or data for various information as described above. Operators may annotate the patient's data with additional information such as the family history or demographics.

The system 100 is configured to determine features embeddings at each node of the coronary graph network that represents the topology. The coronary tree model described above may be converted into a graph that may be represented as a sparse adjacency matrix or via adjacency lists. A sparse matrix only stores non-zero matrix entries. An adjacency list includes an array of nodes and each node points to its adjacency list containing only its neighboring nodes. In addition to representing the topology, each node may include an associated feature vector consisting of the relevant features described above. Each of these vectors may be processed by a machine trained model, for example a fully connected neural network, to obtain a feature embedding associated to each node. In addition to using the features associated to each node, patient-level data may also be included. The graph formulated in this manner may then be processed via a graph neural network.

A graph is a type of data structure that contains nodes and edges. Graph Neural Networks (GNN) are a type of neural networks capable of working with a graph data structure. GNNs are used in predicting nodes, edges, and graph-based tasks. In an embodiment, the graph neural network architecture employs a message-passing mechanism to aggregate, process and pass information between the nodes of the graph. Due to the sparse topology of the graph, applying the message passing method directly is inefficient because information needs to be passed through many nodes to be able to characterize global features of the coronary tree. This issue can be addressed in one of two manners: adding virtual edges connecting nodes pertaining to the same coronary segment and/or generating a higher-level nested graph where each node is representing a coronary segment from the original graph, by computing its embedding based on the embeddings of the underlying nodes pertaining to the coronary segment. In a similar fashion, a global node that aggregates information from the entire coronary tree can be generated.

The graph neural network is used to predict information at a node level, a segment level, and/or a coronary tree level. The graph is input into the graph neural network, which returns an output graph with the same number of nodes, edges, and edge connectivity, but with updated edge, node, and global features. All of the output features are conditioned on the input features according to the graph structure, and are fully differentiable. A classifier may be used to interpret the output, for example, by providing a risk score or other metrics. For example, the output of the graph neural network may be a risk score defined at lesion or patient level, assessing the risk of rupture, clot formation, erosion over a certain time frame.

The graph neural network is trained using ground truth labels that include, for example, follow-up data collected from patient which have suffered plaque ruptures. Since this type of data is very sparse and expensive to collect, the model may be first pre-trained on a large data set using vulnerable plaque indicators, for example including a maximum wall strain, plaque types prone to rupture (spotty calcification, thin cap fibroatheroma, napkin-ring sign, low attenuation plaque), abnormal wall shear stress or abnormal values for other hemodynamic features, and/or other features. Once the graph neural network is pretrained, the graph neural network may be fine-tuned on a smaller dataset where follow-up data is available. Multiple measures, such as hemodynamic measures, QCA scores, plaque vulnerability scores etc. may be learned using a multi-task learning methodology, allowing the predicted measurements to be more consistent, and improving the training behavior due to more supervision signal being provided. To generate ground truth values for the hemodynamic measures, the system may generate a large synthetic database of coronary trees, e.g., using population average values and then run a physics based model on the coronary tree data, e.g. a reduced-order model.

The system 100 is configured to output the predictions of the graph neural network, for example, using a user interface. The user interface may include a display 115 as an output device. Other outputs may be used, such as an interface, network connector, or printer. The output is configured to output the risk of rupture or other risk related to plaque for the patient. The display is a monitor, LCD, projector, plasma display, CRT, printer, or other now known or later developed device for outputting visual information. The display receives images, graphics, text, quantities, or other information from the processor, memory, or medical imaging device 130. The output may be one or more values describing the assessment of vulnerable plaque for the patients or a visual depiction thereof. For example, one or more medical images are displayed that describe the vulnerable plague. The images are of a region of the patient. In one embodiment, the images are of a vessel or vessel tree. The locations of the plaque located by the image processor are highlighted or displayed with other tissue or fluid without highlighting. The image includes an indication, such as a graphic or colorization, of the risk for each plaque or plaque in general for the patient. Alternatively, or additionally, the image includes a quantity based on the classification, such as the risk score value. The quantity may be displayed as the image without the medical image representation of the patient.

In an embodiment, the system 100 may further be configured to generate or support a clinical decision that not only estimates the risk of plaque rupture at different timepoint during the clinical workflow, but also provides procedural indications that allow for minimizing the risk, given all patient-specific data. The system may be configured to provide multiple assessments at different time. For example, a first assessment may be performed after CCTA. The assessment may be updated during the cathlab exam where more data becomes available. The assessment may be further updated after the cathlab exam, especially if PCI was performed for some lesions, i.e., the assessment is performed under post-PCI conditions.

The system may suggest one or more approaches for treating vulnerable coronary plaques. The approaches may include suggesting certain medications. For example, statins, antiplatelet drugs, and anticoagulants may be prescribed to reduce the risk of plaque rupture and blood clot formation. The system may suggest lifestyle modifications such as encouraging a healthy diet, regular exercise, and smoking cessation. CABG and PCI may also be suggested by the system. In addition, or alternatively, the proposed methods may also be employed for predicting patient outcome, given a pre-specified time interval.

Figure 4 depicts an example method for assessing vulnerable plague. The acts are performed by the system of Figure 1, Figure 2, other systems, a workstation, a computer, and/or a server. The acts are performed in the order shown (e.g., top to bottom) or other orders.

In act A110, a medical imaging device 130 scans the patient. A medical image or dataset is acquired. The medical image is a frame of data representing the patient. The data may be in any format. While the terms "image" and "imaging" are used, the image or imaging data may be in a format prior to actual display of the image. For example, the medical image may be a plurality of scalar values representing different locations in a Cartesian or polar coordinate format different from a display format. As another example, the medical image may be a plurality red, green, blue (e.g., RGB) values output to a display for generating the image in the display format. The medical image may be currently or previously displayed image in the display or other format. The image or imaging is a dataset that may be used for imaging, such as scan data representing the patient.

Any type of medical image may be used. In one embodiment, the medical image is a computed tomography (CT) image acquired with a CT system 130. For example, a CT dataset may be used for detecting vessels. For CT, the raw data is reconstructed into a three-dimensional representation. Coronary computed tomography angiography (also called coronary CT angiography or CCTA) uses an injection of iodine-containing contrast material and CT scanning to examine the arteries that supply blood to the heart and determine whether they have been narrowed. Plaque is made of various substances such as fat, cholesterol, and calcium that deposit along the inner lining of the arteries. Plaque, which builds up over time, can reduce or in some cases completely block blood flow. Patients undergoing a CCTA scan receive an iodine-containing contrast material as an intravenous (IV) injection to ensure the best possible images of the heart blood vessels.

The images generated during a CT scan can be reformatted to create three-dimensional (3D) images that may be viewed on a monitor, printed on film or by a 3D printer, or transferred to electronic media. Other medical imaging modalities for acquiring the scan data include X-ray angiography (XA), optical coherence tomography (OCT), ultrasound (US), intra-vascular ultrasound (IVUS), and near-infra red spectroscopy (NIRS). Intra-coronary imaging data (IVUS, OCT) may also be employed to extract information for assessing plaque vulnerability. The medical image data or scan data represents tissue of the patient. Alternatively, the medical image represents flow, velocity, or fluids within the patient. In other embodiments, the data represents both flow and structure. The medical data represents a one, two, or three-dimensional region of the patient. For example, the medical data represents an area or slice of the patient. Values are provided for each of multiple locations distributed in two or three dimensions. The medical data is acquired as a frame of data. The frame of data represents the scan region at a given time or period. The dataset may represent the area or volume over time, such as providing a 4D representation of the patient. The medical image or dataset is acquired by a scan of the patient. The acquisition occurs as part of the scan. Alternatively, the acquisition is from storage or memory, such as acquiring a previously created dataset from a PACS. In an embodiment, Intra-coronary imaging data (IVUS, OCT) may also be employed to extract information for assessing plaque vulnerability.

In an embodiment, rather than relying solely on data from a single timepoint or a single coronary exam, longitudinal data may be incorporated to enable a more comprehensive approach: advanced features that capture the dynamic evolution of the coronary arteries, or of the plaques within the coronary arteries, such as tracking changes in plaque size, composition, and other characteristics over time, may be included (based on all available imaging exams). Additionally, the overall progression of the patient's health state throughout their medical history may provide other critical data to improve the predictive model performance based on a deeper patient understanding, enhancing its accuracy.

In an embodiment, the system further acquires patient-level data that is not linked to a specific location but rather refers to properties of the patient. This type of data may include information such as the presence of other pathologies linked to coronary artery disease in the patient such as AV stenosis, etc. The patient-level data may also include patient demographics, family history, a calcium score, a level of plague burden, lab results, a stress test, among other patient-level data.

At A120, the system 100 generates a coronary tree model 300 of coronary centerlines 310 of a patient, the coronary tree model comprising a plurality of nodes 320 that represent locations in the coronary tree model and edges that represent the relationships between nodes. The 3D centerline model is based on individual centerline points as depicted in Figure 3 described above. A modeling algorithm for coronary arteries may be used to generate the coronary tree model. In an embodiment, the image data is segmented, and one or more machine learning models or algorithms are used to identify the vessels and centerlines.

The system 100 may be configured to segment the CT data using a segmentation model. The CT data is input into a segmentation model that is configured to output a segmented mask when inputting CT data. Any method for segmentation may be used. For example, segmentation may be thresholding-based, region-based, shape-based, model based, neighboring based, and/or machine learning-based among other segmentation techniques. Thresholding-based methods segment the image data by creating binary partitions based on image attenuation values, as determined by the relative attenuation of structures on the images. Region-based segmentation compares one pixel in an image to neighboring pixels, and if a predefined region criterion (e.g., homogeneity) is met, then the pixel/ voxel is assigned to the same class as one or more of its neighbors. Shape-based techniques use either an atlas-based approach or a model-based approach to find a lumen boundary. Model-based methods use prior shape information, similar to atlas-based approaches; however, to better accommodate the shape variabilities, the model-based approaches fit either statistical shape or appearance models of the heart to the image by using an optimization procedure. Vessels and centerlines of the vessels may be derived from the segmented mask, for example, using a classifier.

In an embodiment, instead of using a 3D geometry reconstructed from multiple coronary angiography acquisitions, a 2.5D geometry is derived by matching 2D geometries extracted from each acquisition. Instead of using a 3D centerline-based representation of coronary arteries, the system may instead use a 3D volumetric reconstruction of centerlines from CTA or X-ray angiography. Features computed at Act A130 described below may then be computed in 2D(+time) and aggregated in a 3D(+time) volumetric grid for X-ray angiography. Similarly, features from CTA images can be extracted in 3D(+time) and efficiently fused with the features obtained from X-ray angiography. This approach may make it easier to fuse information from multiple image modalities (CTA, X-ray, IVUS/OCT) by the help of a 3D volumetric grid.

At A130, at each location (node) in the coronary tree model, a set of features fi(x,t) is determined / computed / derived from the image data and patient data, where fi refers to the feature, x refers to the 3D location, and t refers to the time. Some of the features may be constant in time. Some of the features may be obtained by running other AI models or algorithms on the available input data. Anatomical features of a vessel or plaque and/or a morphological feature of the plaque may be extracted from the scan of the patient by the medical imaging device 130 at A110. Other features extracted include parameters for one or more abnormalities of the vessel structure. Abnormal morphology may be characterized by characteristics of calcification, characteristics of the plaque (e.g., fibrous tissue, lipid tissue, necrotic tissue, calcified tissue), characteristics of thrombus, characteristics of diffuse disease, presence of total or sub-total occlusion, presence of myocardial bridging (superficial and/or deep), congenital anomalies of coronary arteries (e.g., anomalous origin of a coronary artery from an abnormal sinus of Valsalva with an inter-arterial course between the great arteries, anomalous origin of one coronary artery from the pulmonary trunk, or others), aneurysmal dilatation and superimposed atherosclerosis, "high take off' coronary artery (e.g., the ostium is several millimeters above the sino-tubular junction (the artery may have a sharp downward angle and runs partially through the aortic wall), myocardial bridging: superficial and deep, coronary fistula, coronary artery dissection, coronary vasculitis (e.g., rheumatoid arthritis, systemic lupus erythematosus (SLE), or Behçet's disease, Kawasaki disease, polyarteritis nodosa, and/or persisting (post) inflammatory aneurysms), fibromuscular dysplasia, coronary micro embolization, and/or left or right dominance. Additional, different, or fewer abnormality features may be used.

Functional features representing operation of the vessel structure may be extracted. Functional information includes functional imaging, such as measures of uptake, or other operational information, such as contrast agent measures. For the training data, the functional features may be determined from simulation, synthetically created images, modeling, and/or other representation of the operation of the vessel.

In addition to anatomic and morphological features from medical images or synthetic representation of a vessel tree, functional features may also be extracted. For example, data from a perfusion scan or other medical imaging scan (e.g., single photon emission computed tomography (SPECT), positron emission tomography (PET), or perfusion imaging) may also be used to extract features such as metrics characterizing relative and/or absolute tissue perfusion in each coronary territory at rest and/or during stress. As another example, angiographic data may characterize contrast agent propagation. Some features characterize the flow of contrast at a given location, such as the time-to-peak tracer concentration, and splits across different daughter vessels at a bifurcation.

A comprehensive list of relevant features is provided below. r(x,t): time-varying lumen radius at each centerline location. r_{ref}(x,t): time-varying reference (i.e. healthy) lumen radius at each centerline location. r_{outer_wall}(x,t): time-varying outer wall radius at each centerline location. It allows for the assessment of positive remodeling. Vulnerable plaque can cause outward bulging of the artery wall, which is known as positive remodeling. This can be seen as an increase in the diameter of the artery segment containing the plaque compared to normal adjacent segments. curvature(x,t): time-varying curvature value at each centerline location. stent(x): binary variable specifying whether a stent is present at each centerline location. Alternatively, an integer type may be used since stents may overlap, and at a given centerline location multiple stents may be present. label(x): integer variable specifying the coronary segment to which the current location pertains. stenosis(x): may be an integer variable specifying a unique id of the stenosis or a floating point variable specifying the stenosis probability at this location. contrast(x,t): integer variable specifying the contrast intensity at this location, which typically varies in time. CABG(x): binary variable specifying whether each centerline location is part of a bypass graft. collateral(x): binary variable specifying whether each centerline location is part of a collateral artery. CTO(x): binary variable specifying if a CTO is encountered at a centerline location(typically a leaf node). myocardialBridging(x): binary variable specifying whether myocardial bridging is observed at a centerline location. Additional similar variables may be defined for other coronary anomalies (e.g., aneurysm). artefact(x): binary variable specifying whether an image artefact is observed at a centerline location (e.g., motion artefact, vessel overlap, etc.). angulation(x): floating point variable specifying the bifurcation angulation at a centerline location (0 if no bifurcation is present). plaqueType(x): integer variable specifying the plaque type: fibrous, fatty, calcified, mixed, etc. Such information may be extracted from angiography (limited) or from CCTA (rich). materialProperties(x): in case PCCT is available, the multi-energy capabilities of PCCT are exploited, which allow for a better characterization of tissue properties and material composition. radiomicFeatures(x). plaquePattern(x): integer variable describing the presence of various plaque patterns indicative of vulnerable plaques: spotty calcification: vulnerable plaque may have small areas of calcification within the plaque, which appear as small, discrete spots on CT images. thin cap fibroatheroma. napkin-ring sign: the napkin-ring sign is a feature seen on CT images that may be indicative of vulnerable plaque. It appears as a ring of high attenuation around a low attenuation core, resembling a napkin ring. This sign suggests that the plaque has a large lipid-rich core and a thin fibrous cap. eccentricity: vulnerable plaque often has an irregular or eccentric shape, with the plaque material located mainly on one side of the vessel wall. low attenuation plaque: vulnerable plaque typically has a large lipid-rich core, which has a lower attenuation value on CT images compared to surrounding tissue. This can appear as a soft, low-density area within the wall of the artery. regionalWallMotion(x): may be derived from angiography, echocardiography, or multi-phase CCTA. downstreamMyocardialVolume(x): myocardial volume subtended by the coronary tree downstream from a given location x

Computed features may also include: FFR(x), IFR(x), restPdPa(x), etc : hemodynamic features computed or predicted for the coronary tree. WSS(x,t), OSI(x,t): location and time-dependent wall shear stress, oscillatory shear index. Previous studies have shown the high / low stress are linked with plaque evolution and or rupture. Q(x,t): time-varying flow rate. V(x,t): time-varying velocity.

Rather than relying solely on data from a single timepoint or a single coronary exam, longitudinal data may be incorporated to enable a more comprehensive approach: advanced features that capture the dynamic evolution of the coronary arteries, or of the plaques within the coronary arteries, such as tracking changes in plaque size, composition, and other characteristics over time, could be included (based on all available imaging exams). Additionally, the overall progression of the patient's health state throughout their medical history may provide other critical data to improve the predictive model performance based on a deeper patient understanding, enhancing its accuracy.

The derived features and computed features may be provided using a machine learned network or model. Different networks / models may be specifically configured for each respective task of deriving or computing a feature. Different networks and configurations may be used. For example, a DenseNet or other network arrangements may also be used for the trained networks or other trained networks described above for segmentation, classification, or analysis. A DenseNet connects each layer in a network to every other layer in a feed-forward fashion. For each layer in the DenseNet, the feature-maps of all preceding layers are used as inputs, and the output feature-map of that layer is used as input into all subsequent layers. In the DenseNet, for each layer, the feature maps of all preceding layers are used as inputs, and its own feature maps are used as inputs into all subsequent layers. To reduce the size of the network, the DenseNet may include transition layers. The layers include convolution followed by average pooling. The transition layers reduce height and width dimensions but leave the feature dimension the same. The neural network may further be configured as a U-net. The U-Net is an autoencoder in which the outputs from the encoder-half of the network are concatenated with the mirrored counterparts in the decoder-half of the network. Skip connections prevent the middle of the network from becoming a bottleneck.

Other deep architectures that may be used include convolutional neural network (CNN) or deep belief nets (DBN), but other deep networks may be used. CNN learns feed-forward mapping functions while DBN learns a generative model of data. In addition, CNN uses shared weights for all local regions while DBN is a fully connected network (e.g., including different weights for all regions of an image). The training of CNN is entirely discriminative through back-propagation. DBN, on the other hand, employs the layer-wise unsupervised training (e.g., pre-training) followed by the discriminative refinement with back-propagation if necessary. In an embodiment, the arrangement of the trained network is a fully convolutional network (FCN). Alternative network arrangements may be used, for example, a 3D Very Deep Convolutional Networks (3D-VGGNet). VGGNet stacks many layer blocks containing narrow convolutional layers followed by max pooling layers. A 3D Deep Residual Networks (3D-ResNet) architecture may be used. A Resnet uses residual blocks and skip connections to learn residual mapping.

At act A140, the system determines a feature embedding associated with each node from the patient-level data and the respective set of features for the respective node. In addition to representing the topology, each node can have an associated feature vector consisting of the features described previously. Each of these vectors can be processed by a fully connected neural network to obtain a feature embedding associated to each node. When computing these embeddings, in addition to using the features associated to each node, patient-level data from A140 may also be included. The coronary tree model of Act A120 may be presented as a graph which can be represented as a sparse adjacency matrix or via adjacency lists. Adjacency lists describe the connectivity of edge between nodes as a tuple in an entry of an adjacency list.

At act A150, the system inputs the feature embeddings into a graph neural network (GNN) and predicts diagnostic information at a node level, a segment level, and/or a coronary tree level based on the output of the graph neural network. The graph neural network (GNN) is an optimizable transformation on all attributes of the graph (nodes, edges, global context) that preserves graph symmetries (permutation invariances). The GNN uses a graph-in, graph-out architecture meaning that the model accepts the coronary tree as input, with the features embeddings loaded into its nodes, edges, and global-context, and progressively transform the embeddings, without changing the connectivity of the input graph. The main unit of computation is the GN block, a graph-to-graph module that takes the graph as input, performs computations over the structure, and returns the graph as output. Entities are represented by the graph's nodes, relations by the edges, and system-level properties by global attributes. Predictions may be made by the GNN by using pooling within the GNN's layer, in order to make the learned embeddings aware of graph connectivity. This is done using message passing, where neighboring nodes or edges exchange information and influence each other's updated embeddings. The proposed architecture employs a message-passing mechanism to aggregate, process and pass information between the nodes of the graph. Message passing works in three steps. For each node in the graph, all the neighboring node embeddings (or messages) are gathered. The messages are aggregated via an aggregate function (like sum). The pooled messages are passed through an update function, for example a neural network. Just as pooling can be applied to either nodes or edges, message passing can occur between either nodes or edges.

Due to the sparse topology of the graph, applying the message passing method directly may be inefficient on its own because information needs to be passed through many nodes to be able to characterize global features of the coronary tree. This issue is addressed in two manners. Virtual edges are added that connect nodes pertaining to the same coronary segment or generating a higher-level nested graph where each node represents a coronary segment from the original graph. Embeddings may be computed based on the embeddings of the underlying nodes pertaining to the coronary segment. In a similar fashion to the second approach, a global node that aggregates information from the entire coronary tree can be generated.

By applying the proposed graphnet architecture on the obtained nested graph, information may be predicted at node level, segment level and coronary tree level. In an embodiment, the output of the GNN is a prediction, such as a risk score. The risk of rupture is predicted in one embodiment. In another embodiment, risk stratification is provided. For example, both risk stratification and plaque vulnerability assessment are calculated from or as the risk score for the patient in a coronary artery disease analysis. Vulnerable coronary plaque refers to a type of plaque in the coronary arteries that is more likely to rupture or cause a blood clot, which can lead to a heart attack. Hence, the output of the model may be a risk score defined at lesion or patient level, assessing the risk of rupture, clot formation, erosion over a certain time frame.

In an embodiment, a clinical decision support may be provided that not only estimates the risk of plaque rupture at different timepoint during the clinical workflow, but also provides procedural indications that allow for minimizing the risk, given all patient-specific data. A first assessment may be performed after CCTA. The assessment may be updated during the cathlab exam where more data becomes available. The assessment may be further updated after the cathlab exam, especially if PCI was performed for some lesions, i.e., the assessment is performed under post-PCI conditions.

There are several approaches to treating vulnerable coronary plaques, including: Medications: statins, antiplatelet drugs, and anticoagulants may be prescribed to reduce the risk of plaque rupture and blood clot formation. Lifestyle modifications: Encouraging a healthy diet, regular exercise, and smoking cessation can help prevent the progression of vulnerable plaques. Alternatively, the proposed methods may also be employed for predicting patient outcome, given a pre-specified time interval.

In an embodiment, ground truth labels for training the model include follow-up data collected from patient that have suffered plaque ruptures. Since this type of data is very sparse and expensive to collect, the model may be first pre-trained on a large data set using vulnerable plaque indicators such as maximum wall strain, plaque types prone to rupture (spotty calcification, thin cap fibroatheroma, napkin-ring sign, low attenuation plaque), abnormal wall shear stress or abnormal values for other hemodynamic features, etc. Once the model is pretrained, it may be fine-tuned on a smaller dataset where follow-up data is available. In addition, multiple measures, such as hemodynamic measures, QCA scores, plaque vulnerability scores etc. may be learned using a multi-task learning methodology, allowing the predicted measurements to be more consistent, and improving the training behavior due to more supervision signal being provided. To generate ground truth values for the hemodynamic measures, the system may generate a large synthetic database of coronary trees, e,g., using population average values and run a physics based model on the coronary tree data, e.g. a reduced-order model.

It is to be understood that the elements and features recited in the appended claims may be combined in different ways to produce new claims that likewise fall within the scope of the present invention. Thus, whereas the dependent claims appended below depend on only a single independent or dependent claim, it is to be understood that these dependent claims may, alternatively, be made to depend in the alternative from any preceding or following claim, whether independent or dependent, and that such new combinations are to be understood as forming a part of the present specification.

While the present invention has been described above by reference to various embodiments, it may be understood that many changes and modifications may be made to the described embodiments. It is therefore intended that the foregoing description be regarded as illustrative rather than limiting, and that it be understood that all equivalents and/or combinations of embodiments are intended to be included in this description. Independent of the grammatical term usage, individuals with male, female or other gender identities are included within the term.

The following is a list of non-limiting illustrative embodiments disclosed herein:
Illustrative embodiment 1. A method for vulnerable plaque assessment and outcome prediction in coronary artery disease, the method comprising: acquiring medical imaging data of a patient; generating a coronary tree model of coronary centerlines of a patient from the medical imaging data, the coronary tree model comprising a plurality of nodes that represent locations in the coronary tree model; determining a feature embedding associated with each node from a plurality of features derived from the medical imaging data; inputting the feature embeddings into a graph neural network; and outputting an assessment at a node level, a segment level, and/or a coronary tree level for vulnerable plaque based on the output of the graph neural network.
Illustrative embodiment 2. The method of Illustrative embodiment 1, wherein the medical imaging data is coronary computed tomography angiography (CCTA) data.
Illustrative embodiment 3. The method of Illustrative embodiment 1, further comprising: acquiring patient level data, wherein the patient level data is not specific to any location in the coronary tree model, wherein the patient level data is input into the graph neural network.
Illustrative embodiment 4. The method of Illustrative embodiment 3, wherein the patient level data comprises at least one of a presence of other pathologies linked to coronary artery disease, patient demographics, patient history, family history, a calcium scored, an overall plaque burden, lab results, or results of a stress test.
Illustrative embodiment 5. The method of Illustrative embodiment 1, wherein generating the coronary tree model comprises segmenting the medical imaging data using a thresholding method, wherein the coronary tree model includes all locations with a diameter larger than a given threshold of 1.0 mm.
Illustrative embodiment 6. The method of Illustrative embodiment 1, wherein generating the coronary tree model comprises deriving a 2.5D geometry by matching 2D geometries extracted from each acquisition.
Illustrative embodiment 7. The method of Illustrative embodiment 1, wherein determining the feature embeddings comprises: defining a set of features fi(x,t) for each respective node of the plurality of nodes, where fi refers to the feature, x refers to a 3D location in the coronary tree model, and t refers to a time; and inputting the set of features into a machine trained network configured to output a feature embedding for each respective node.
Illustrative embodiment 8. The method of Illustrative embodiment 7, wherein values for the set of features changes depending on a state of the patient.
Illustrative embodiment 9. The method of Illustrative embodiment 7, wherein multiple instances of certain features are averaged to determine the set of features.
Illustrative embodiment 10. The method of Illustrative embodiment 1, wherein the graph neural network uses a message-passing mechanism to aggregate, process and pass information between the nodes of the graph neural network.
Illustrative embodiment 11. The method of Illustrative embodiment 10, wherein virtual edges connecting nodes pertaining to the same coronary segment are added to the graph neural network for the message-passing mechanism.
Illustrative embodiment 12. The method of Illustrative embodiment 1, wherein the assessment comprises a risk score defined at lesion or patient level, assessing the risk of rupture, clot formation, or erosion over a certain time frame.
Illustrative embodiment 13. The method of Illustrative embodiment 1, further comprising: assessing the vulnerable plaque of the patient based on the assessment; and providing procedural indications that allow for minimizing a risk to the patient of the vulnerable plaque.
Illustrative embodiment 14. The method of Illustrative embodiment 13, wherein the vulnerable plaque of the patient is assessed after the medical imaging data is acquired, wherein the assessment is updated during a cathlab exam, and wherein the assessment is further updated after the cathlab exam.
Illustrative embodiment 15. A system for vulnerable plaque assessment, the system comprising: a medical imaging system configured to acquire medical imaging data of a patient; an image processing system configured to generate a coronary tree of coronary centerlines of a patient from the medical imaging data, the coronary tree model comprising a plurality of nodes that represent locations in the coronary tree model, the image processing system further configured to compute or derive one or more features associated with each node from a plurality of features derived from the medical imaging data, the image processing system further configured to determine a feature embedding for each node based on the one or more features and input the feature embeddings into a graph neural network configured to generate a vulnerable plaque assessment; and an output interface configured to provide the vulnerable plaque assessment.
Illustrative embodiment 16. The system of Illustrative embodiment 15, wherein each node of the graph neural network is associated with a feature vector consisting of the one or more features, wherein the feature vectors are processed by a fully connected neural network to obtain the feature embedding associated to each node.
Illustrative embodiment 17. The system of Illustrative embodiment 15, wherein the one or more features comprise a set of features fi(x,t) for each respective node of the plurality of nodes, where fi refers to the feature, x refers to a 3D location in the coronary tree model, and t refers to a time.
Illustrative embodiment 18. The system of Illustrative embodiment 15, wherein generating the coronary tree model comprises segmenting the medical imaging data using a thresholding method, wherein the coronary tree model includes all locations with a diameter larger than a given threshold of 1.0 mm.
Illustrative embodiment 19. The system of Illustrative embodiment 15, wherein the medical imaging data is coronary computed tomography angiography (CCTA) data.
Illustrative embodiment 20. A non-transitory computer implemented storage medium that stores machine-readable instructions executable by at least one processor, the machine-readable instructions comprising: generating a coronary tree model of coronary centerlines of a patient from medical imaging data, the coronary tree model comprising a plurality of nodes that represent locations in the coronary tree model; determining a feature embedding associated with each node from a plurality of features derived from the medical imaging data; inputting the feature embeddings into a graph neural network; and outputting an assessment at a node level, a segment level, and/or a coronary tree level for vulnerable plaque based on the output of the graph neural network.

## Claims

1. A method for vulnerable plaque assessment and outcome prediction in coronary artery disease, the method comprising:
acquiring (A110) medical imaging data of a patient (210);
generating (A120) a coronary tree model (300) of coronary centerlines (310) of the patient (210) from the medical imaging data, the coronary tree model (300) comprising a plurality of nodes that represent locations in the coronary tree model (300);
determining (A140) a feature embedding associated with each node (320) from a plurality of features derived from the medical imaging data;
inputting (A150) the feature embeddings into a graph neural network; and
outputting (A150) an assessment at a node level, a segment level, and/or a coronary tree level for vulnerable plaque based on the output of the graph neural network.

2. The method of claim 1, further comprising:
acquiring patient level data, wherein the patient level data is not specific to any location in the coronary tree model (300), wherein the patient level data is input into the graph neural network.

3. The method of claim 2, wherein the patient level data comprises at least one of a presence of other pathologies linked to coronary artery disease, patient demographics, patient history, family history, a calcium scored, an overall plaque burden, lab results, or results of a stress test.

4. The method of any one of claims 1 - 3, wherein generating the coronary tree model (300) comprises
segmenting the medical imaging data using a thresholding method, wherein the coronary tree model (300) includes all locations with a diameter larger than a given threshold of 1.0 mm; and/or
deriving a 2.5D geometry by matching 2D geometries extracted from each acquisition.

5. The method of any one of claims 1 - 4, wherein determining the feature embeddings comprises:
defining (A130) a set of features fi(x,t) for each respective node (320) of the plurality of nodes (320), where fi refers to the feature, x refers to a 3D location in the coronary tree model (300), and t refers to a time; and
inputting the set of features into a machine trained network configured to output a feature embedding for each respective node (320).

6. The method of claim 5, wherein
values for the set of features changes depending on a state of the patient (210); and/or
multiple instances of certain features are averaged to determine the set of features.

7. The method of any one of claims 1 - 6, wherein the graph neural network uses a message-passing mechanism to aggregate, process and pass information between the plurality of nodes (320) of the graph neural network.

8. The method of claim 7, wherein virtual edges connecting nodes pertaining to the same coronary segment are added to the graph neural network for the message-passing mechanism.

9. The method of any one of claims 1 - 8, wherein the assessment comprises a risk score defined at lesion or patient level, assessing the risk of rupture, clot formation, or erosion over a certain time frame.

10. The method of any one of claims 1 - 9, further comprising:
assessing the vulnerable plaque of the patient (210) based on the assessment; and
providing procedural indications that allow for minimizing a risk to the patient (210) of the vulnerable plaque.

11. The method of claim 10, wherein the vulnerable plaque of the patient (210) is assessed after the medical imaging data is acquired, wherein the assessment is updated during a cathlab exam, and wherein the assessment is further updated after the cathlab exam.

12. A system for vulnerable plaque assessment, the system comprising:
a medical imaging system (130) configured to acquire medical imaging data of a patient (210);
an image processing system (105) configured to generate a coronary tree of coronary centerlines (310) of the patient (210) from the medical imaging data, the coronary tree model (300) comprising a plurality of nodes that represent locations in the coronary tree model (300), the image processing system (105) further configured to compute or derive one or more features associated with each node from a plurality of features derived from the medical imaging data, the image processing system (105) further configured to determine a feature embedding for each node based on the one or more features and input the feature embeddings into a graph neural network configured to generate a vulnerable plaque assessment; and
an output interface (115) configured to provide the vulnerable plaque assessment.

13. The system of claim 12, wherein
each node of the graph neural network is associated with a feature vector consisting of the one or more features, wherein the feature vectors are processed by a fully connected neural network to obtain the feature embedding associated to each node; and/or
the one or more features comprise a set of features fi(x,t) for each respective node of the plurality of nodes, where fi refers to the feature, x refers to a 3D location in the coronary tree model (300), and t refers to a time; and/or
generating the coronary tree model (300) comprises segmenting the medical imaging data using a thresholding method, wherein the coronary tree model (300) includes all locations with a diameter larger than a given threshold of 1.0 mm; and/or
the system comprises means for carrying out the method steps according to the method of any one of claims 1 - 11.

14. The method of any one of claims 1 - 11 or the system of claim 12 or 13, wherein the medical imaging data is coronary computed tomography angiography, CCTA, data.

15. A non-transitory computer implemented storage medium that stores machine-readable instructions, which when executed by at least one processor, cause the at least one processor to perform the method steps according to the method of any one of claims 1 - 11 or 14.
